# EUROPEAN PATENT SPECIFICATION

(11) **EP 1 326 546 B1**
(45) Date of publication and mention of the grant of the patent: **14.01.2009**
(21) Application number: 01987680.4
(22) Date of filing: 19.10.2001
(51) Int. Cl.: A61B 17/34

(54) **ADAPTOR CAP FOR TROCAR ASSEMBLY**
ADAPTER- UND VERSCHLUSSEINHEIT FÜR TROKAREINRICHTUNG
JOINT POUR TROCART FIXE

(30) Priority: 19.10.2000 US 241665 P
(43) Date of publication of application: 16.07.2003
(73) Proprietor: Tyco Healthcare Group LP, Norwalk, Connecticut 06856 (US)
(72) Inventor: SMITH, Robert, C., Hamden, CT 06518 (US)
(74) Representative: HOFFMANN EITLE
(86) International application number: PCT/US2001/045324
(87) International publication number: WO 2002/032481

(56) References cited:
- EP-A- 0 604 748
- DE-U- 29 717 940
- US-A- 5 338 307
- US-A- 5 868 714
- US-A- 6 123 689

## Description

### BACKGROUND

### 1. Technical Field

The present disclosure relates to a mechanism for controlling the operable area of a passageway defined in a valve assembly of a trocar. More particularly, the present disclosure relates to a diameter reduction mechanism that is removably positionable over the passageway of the trocar to restrict the movement of small diameter surgical instruments to a reduced operable area of the passageway without sealing with the trocar and pivot clear of the passageway to accommodate large diameter surgical instruments. US-A-5,868,714 discloses a reducer cap for a trocar assembly, which cap includes a wiper seal of elastic material for maintaining abdominal pressure during use of small diameter surgical tools.

### 2. Background of Related Art

Trocar valve assemblies are configured to provide a fluid tight sealing system before, during, and after a surgical instrument is entered through the trocar during minimally invasive surgical procedures. Sealing systems, such as a valve assembly for a passageway defined by a trocar connected with a cannula include an outer seal which can be fixed or floating, in combination with additional inner seals. Fixed outer seals are limited by their ability to sustain a seal when smaller surgical instruments are moved off-axis, away from and generally parallel to the central longitudinal axis, towards the inside circumference of the cannula. Fixed seals are also limited by their ability to sustain their integrity when small diameter surgical tool angulation is employed. These extreme ranges of motion of smaller diameter surgical instruments within the cannula can create a "cat eye" or crescent shaped gap in the fixed seal that can result in a loss of seal integrity. Additional problems include the ability of a sealing system to be sufficiently flexible to maintain its integrity when both small diameter and large diameter surgical instruments are used.

Devices to restrict the diameter of a passageway in a trocar housing generally require a complex additional mechanism to be positioned on the proximal end of the trocar housing that restricts the range of motion of small surgical instruments and include additional seals or replace seals of the valve assembly with seals configured for small surgical instruments. These diameter reducing devices, however, typically require the disassembly and assembly of the proximal end of the trocar to adjust for the varying sizes of diameter reduction devices.

A continuing need exists for a diameter reducing structure that can limit parallel off-axis as well as angular movements of small diameter surgical instruments and accommodate larger diameter surgical instruments without disassembling the proximal end of the trocar.

For other disclosures of a cannula with a seal see US-A-6,123,689, 5,338,307, DE-U-297 17 940 and EP-A-0 604 748.

### SUMMARY

The present invention is defined in the independent claim presented below. The dependent claims are directed to optional or preferred features.

A diameter reduction mechanism is provided for assisting the valve assembly of a trocar in maintaining a seal. The diameter reduction mechanism includes a housing and a movable limiter. The limiter is diametrically positionable over the passageway to control the parallel off-axis and angular movements of smaller diameter surgical instruments. The limiter includes a locking mechanism and a pivot. The locking mechanism is adapted for use by a surgeon to readily lock the limiter in position over the passageway and to unlock and move the limiter clear of the passageway. Once the limiter is clear of the passageway, larger diameter surgical instruments are freely positionable within the passageway without any further adjustments by the operator.

The invention, together with attendant advantages, will be best understood by reference to the following detailed description of the invention when used in conjunction with the figures below.

### BRIEF DESCRIPTION OF THE DRAWINGS

Preferred embodiments of the presently disclosed diameter reduction structure fixed trocar seal are described herein with reference to the drawings, wherein:
FIG. 1 is a top view of one preferred embodiment of a diameter reduction mechanism for a fixed trocar seal constructed in accordance with the present disclosure;
FIG. 2 is a cross-sectional side view of the diameter reduction mechanism for the fixed trocar seal of FIG. 1 along lines A-A;
FIG. 3 is a cross-sectional side view of the diameter reduction mechanism for the fixed trocar seal of FIG. 1 along lines B-B;
FIG. 4 is a close-up of a partial cross-sectional side view of the diameter reduction mechanism for the fixed trocar seal of FIG. 1 along lines C-C;
FIG. 5 is a close-up of detail D of a pivot assembly of the diameter reduction mechanism for the fixed trocar seal of FIG. 2;
FIG. 6 is a close-up of detail E of the interface between the valve assembly and the diameter reduction mechanism for the fixed trocar seal of FIG. 2; and
FIG. 7 is a perspective view of the top of the diameter reduction mechanism for the fixed trocar seal of FIG. 1.

### DETAILED DESCRIPTION OF THE EMBODIMENTS

The present disclosure contemplates the introduction into a body of a patient a trocar adapted for receiving all types of surgical instruments including clip appliers, graspers, dissectors, retractors, staplers, laser fibers, endoscopes, as well as electrosurgical cutting, coagulating, and ablation devices, and the like. All such objects are referred to herein as "instruments".

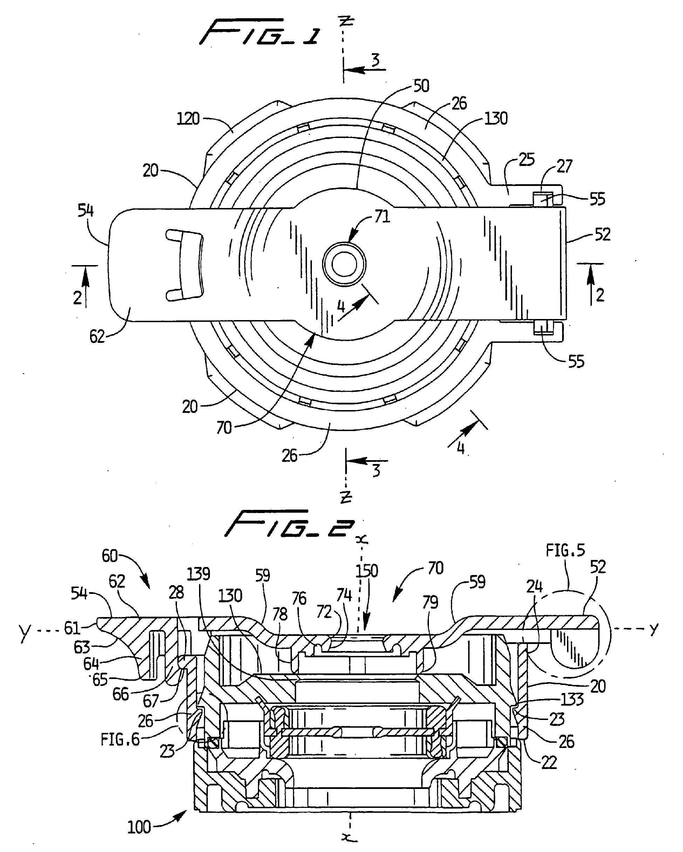

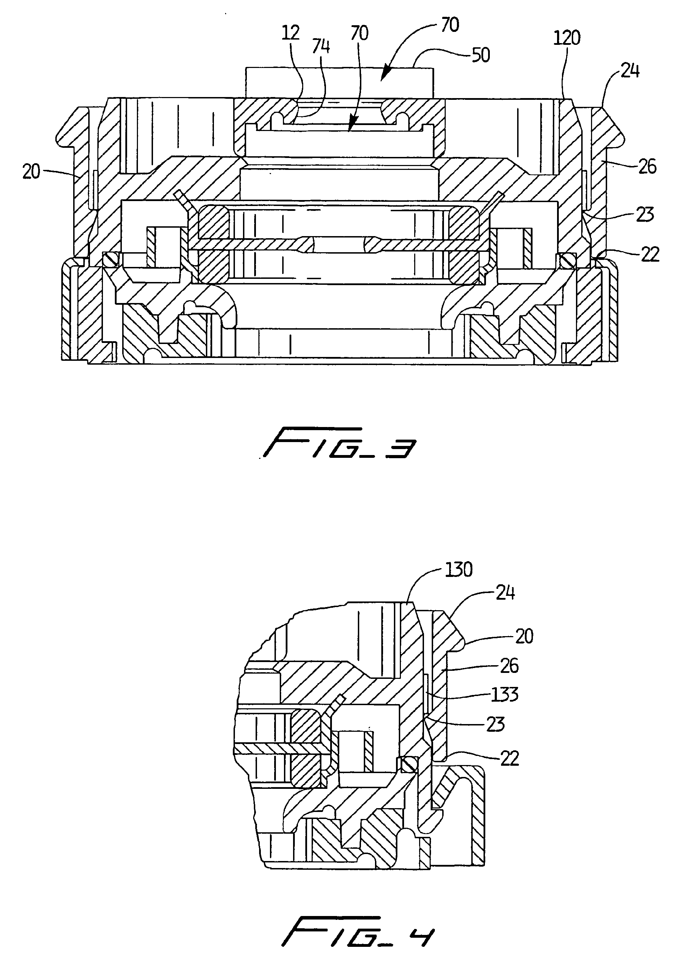

Referring now in specific detail to the drawings in which like referenced numerals identify similar or identical elements throughout the several views, and initially to FIGS. 1-3, a novel diameter reduction mechanism for a fixed trocar seal 10 is shown constructed in accordance with the present disclosure and intended to be configured as an integral portion or an attachment to a conventional trocar assembly 100. Trocar assembly 100 includes valve assembly 120, end cap 130, and cannula (not shown). Trocar assembly 100 defines a passageway 150 aligned with a central longitudinal axis-X. Passageway 150 defines a first operable area. Valve assembly 120 is configured to provide a seal between a cavity formed in the patient and the outside atmosphere during and subsequent to insertion of an instrument through the cannula.

Diameter reduction mechanism for fixed trocar seal 10 includes a housing 20 and a limiter 50. Housing 20, in one preferred embodiment is an integral and proximal portion of valve assembly 120 of trocar 100. In another preferred embodiment, housing 20 is a tubular shaped ring adapted for being removably connected to a proximal end of valve assembly 120. Housing 20 includes a tubular wall 26 having a distal end portion 22 and a proximal end portion 24 aligned with the longitudinal axis-X.

Referring now to FIGS. 2-4 and 6, distal end portion 22 of tubular wall 26 includes an inwardly and radially extending protuberance 23 configured to engagingly mate and lock with an annular lip 133 positioned on end cap 130 of valve assembly 120.

As shown in FIGS. 1, 2 and 5, proximal end portion 24 includes a receptacle 25 having a pair of opposed notches 27 configured for the pivoting of limiter 50 and a diametrically opposed lip 28 positioned on wall 26. Lip 28 has an arcuate shaped portion extending outwardly and radially from wall 26 with a distal facing generally flat portion.

Referring now to FIGS. 1-3 and 5-6, in one preferred embodiment, diameter reduction mechanism 10 in a first position includes housing 20 lockingly engaged with valve assembly 120 and limiter or reduction member 50 extending diametrically over passageway 150 and valve assembly 120. Limiter 50 includes a first end 52 being pivotally connected to a receptacle 25 of housing 20 and a second end 54 being engaged and locked with lip 28. An axis-Z is defined perpendicular to limiter 50 and axis-Y that intersects axis-X.

Limiter 50 has an elongate planar beam shape having a thickness in the direction of axis-X and a width in the direction of axis-Z. The width of limiter 50 can vary, but is approximately less than or equal to the diameter of passageway 150. A hole or opening 71 is centrally positioned on limiter 50 and aligned with the longitudinal axis-X. Limiter 50 also includes an annular recess 59 positioning hole 71 distal to first end 52 and second end 54 such that a proximal side of a rim 72 of hole 71 is positioned approximately equal to or below the proximal edge of valve assembly 120.

In FIGS. 1-3 and 5, first end 52 can include interfaces such as a hinge, a threaded connection, or a slide, such that the limiter 50 can pivot about notch 27 of receptacle 25. In one preferred embodiment first end 54 includes a pair pins 55 perpendicular to axis-Y positionable within notches 27. Pins 55 have a controlled tolerance to fit tightly within notches 27 for a pure rotational hinge type movement or notches 27 can include a predetermined amount of play or bias such that limiter 50 can translate a controlled distance along axis-Y, for example, or pivot angularly within a plane Z-Y formed by the intersection of axes Z and Y.

Referring now to FIGS. 1,2 and 7, second end 54 includes a locking mechanism 60 having a first cantilevered element 62 extending along axis-Y with a second cantilevered element 64 and a third cantilevered element or locking element 66 extending distally therefrom and generally parallel to longitudinal axis-X. A distal end of element 66 includes a radially inwardly extending protuberance 67 configured for engagingly interfacing with lip 28 to lock limiter 50 in the first position. A fillet 63 extends between an edge 61 of element 62 and a distal end edge 65 of element 64. Fillet 63 is ergonomically configured for the positioning of a limb or portion of a limb of a surgeon for the proximal movement or flexing of second end 54 to disengage protuberance 67 from lip 28.

Limiter 50 includes an interior portion 70 having a proximal first rim 72 and a distal second rim 74 defining hole 71 as well as an annular portion 78. Rim 72 has a conical shape with a distally decreasing inside diameter and rim 74 has a conical shape with a distally increasing diameter. The conical shapes of rims 72 and 74 are advantageously configured to support a limited degree of small surgical instrument angulation. Hole 71 is aligned with the longitudinal axis-X in the first position and less than the inside diameter of passageway 150 by a predetermined amount, depending upon the desired application. Thus, hole 71 reduces the first operable area to a second operable area less than the first operable area. It is envisioned that diameter reduction mechanism for fixed trocar seal 10 can be provided in a variety of reducing diameters in a kit configuration having multiple limiters 50 and/or mechanisms 10 and be readily removed and replaced as an assembly without interrupting the integrity of the valve assembly.

As shown in FIGS. 1-3 and 7, interior portion 70 includes an annular channel 76 extending proximally from the distal side of limiter 50. Channel 76 is in apposition with rim 74 and defines rim 74 as a distally extending cantilevered element that provides a controlled degree of flexing or bias by rim 74 to urge instruments towards the central longitudinal axis. Annular portion 78 extends distally from interior portion 70. Annular portion 78 has a distal end 79 configured for seating on a rim 139 of a proximal end of end cap 130. Rim 139 defines a proximal portion of passageway 150 in conjunction with annular portion 78 in the first position. Annular portion 78 defines an inside diameter approximately equivalent to the proximal portion of passageway 150 defined by end cap 130. Thus, limiter 50 in the first position is configured as a cross beam supported on ends 54 and 52 by housing 20 and by the positioning of annular portion 78 in direct contact with rim 139 of end cap 130. Annular portion 78 provides structural support for limiter 50 and it precludes the misalignment of instruments while providing a non-sealing interface with passageway 150.

Limiter 50 is movably positioned on housing 20 such that when the surgeon desires to use instruments larger than the diameter of hole 71, the limiter 50 can be readily pivoted out of the first position over passageway 150 by using the limb or the portion of the limb of the surgeon to pull proximally against fillet 63 or edge 61. When limiter 50 is pivoted clear of passageway 150, diameter reduction mechanism for fixed trocar seal 10 is in a second position. Limiter 50 in the second position, is preferably extending distally and parallel to the longitudinal axis-X, but it may include stops or be biased to a range of positions from extending proximally and generally parallel to the longitudinal axis-X to being parallel to axis-Y, for example.

It is also envisioned that limiter 50 can be suitably configured for translation or angular movement with the X-Y plane such that limiter 50 can include a variable circumference configured to be aligned with axis-X that can be integral to or removably attached to limiter 50 to accommodate varying sized instruments without removing limiter 50 or diameter reduction mechanism for fixed trocar seal 10 from the trocar.

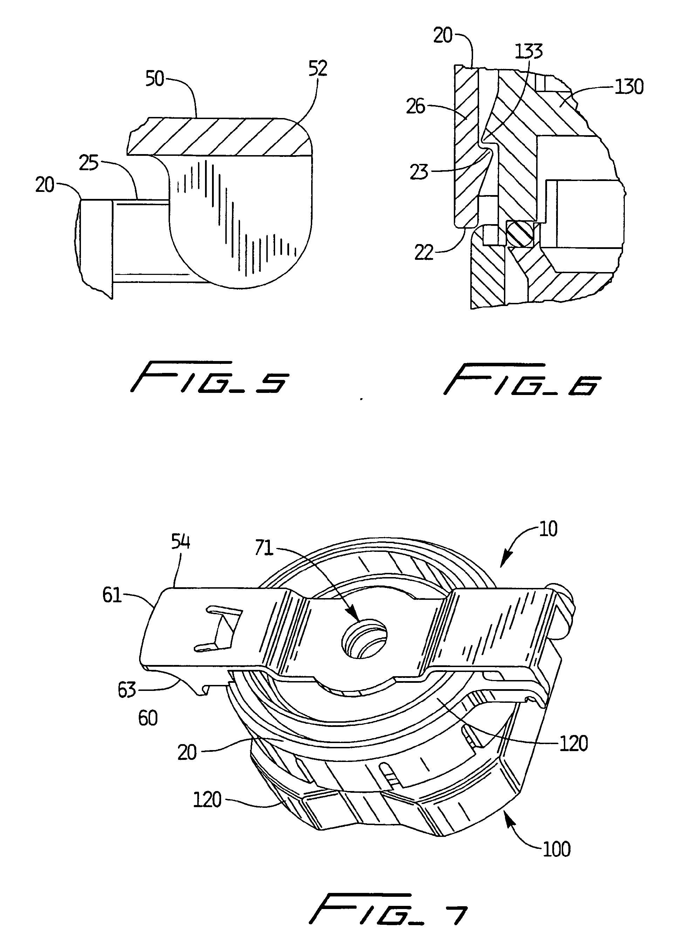

Referring now to FIG. 7, in operation, diameter reduction mechanism for fixed trocar seal 10 is positioned over a proximal end of valve assembly 120 of trocar 100 and forwarded distally to engage and lock housing 20 with valve assembly 120. Moving limiter 50 accommodates the selective changing of the operable area of passageway 150. Limiter 50 in the first position is configured to limit the off axis movements as well as the angulation of small diameter surgical instruments less than or equal to the diameter of hole 71. Limiter 50 is adapted to readily accommodate, with a single movement of edge 61 or fillet 63, the movement of limiter 50 from the first position to the second position for the positioning of larger diameter surgical instruments. Limiter 50 is adapted for being readily replaced to the first position by the limb of the surgeon positioning second end 54 to suitably engage locking mechanism 60 to retain limiter 50 in the first position.

## Claims

1. A trocar seal for use with a trocar assembly, which seal comprises:
a housing (20) adapted for mounting to a trocar assembly (100), the housing including an outer wall defining a longitudinal axis (X), and proximal (24) and distal (22) ends, the housing having an inner transverse wall (26);
an inner seal (10) disposed within the housing in general alignment with the longitudinal axis, the inner seal having an aperture for sealed reception of an elongated object introduced therein; and
a reduction member (50) mounted to the housing and movable relative to the housing between first and second positions, the reduction member having an interior portion defining an opening (71) therethrough, the opening being in general alignment with the aperture of the inner seal when the reduction member is in the first position thereof, and clear of the longitudinal axis in the second position, the interior portion in said first position being dimensioned to minimize offset lateral movement of the elongated object during manipulation of the elongated object when the elongated object is inserted in and sealingly received by the inner seal, the reduction member further including an annular portion (78) disposed distal of the interior portion, the annular portion dimensioned to engage the transverse wall of the housing to support the reduction member when in the first position thereof.

2. The trocar seal of claim 1 including an outer seal peripherally disposed with respect to the inner seal and being dimensioned to define a sealing interface to substantially minimize passage of insufflation gases through the housing.

3. The trocar seal of claim 1, wherein the reduction member includes a rim (72, 74), the rim defining the opening, the rim including a proximal first portion (72) and a distal second portion (74), the proximal first portion defining a conical shape and a distally decreasing inside diameter, the distal second portion defining a conical shape and a distally increasing inside diameter.

4. The trocar seal of claim 3, wherein at least a portion (74) of the rim defining the opening is a cantilevered structure, the cantilevered structure including a bias, the bias configured to urge instruments towards the central longitudinal axis.

5. The trocar seal of claim 1, wherein the housing includes a proximal end portion, the proximal end portion defining a rim (139), the annular portion of the reduction member being adapted for mounting on the rim of the proximal end portion of the housing, the annular portion providing an unsealed connection between the rim of the valve assembly and the opening in the reduction member.

6. The trocar seal of claim 1, wherein a locking mechanism is positioned on the reduction member, the locking mechanism including a distally extending cantilevered element having a distal end portion with a protuberance (23), the protuberance extending inwardly and radially and having a generally flat proximally facing surface adapted for engaging with a lip (133) of the housing, the lip having a distally facing generally flat surface.

7. The trocar seal of any one of the preceding claims wherein
the housing includes a tubular wall and is mounted on a proximal end of a trocar valve assembly, the valve assembly having at least one fixed seal including the inner seal and defining a longitudinal passageway, the longitudinal passageway defining a first operable area;
the reduction member having a first end, an opposing second end, and an interior portion, the first end being pivotally connected to the housing, the second end having a locking mechanism for engaging with the housing, the reduction member in said first position being positioned over the first operable area of the passageway with the second end locked with the housing, the pivotal connection permitting movement and in said second position being positioned clear of the first operable area of the passageway, the reduction member and housing providing an unsealed interface with the valve assembly;
a rim (13a) positioned on the interior portion of the reduction member, the rim defining a hole aligned with the passageway, the hole defining a second operable area less than the first operable area of the passageway.

8. The trocar seal of claim 7, wherein the housing includes a receptacle having a pair of notches (27) and the first end of the reduction member includes a pair of opposing linearly aligned pins (55), the pins being configured and dimensioned for positioning in the notches of the receptacle and permitting movement of the reduction member.

9. The trocar seal of claim 8, wherein the receptacle and the pins are configured to support the translation of the reduction member in a plane aligned with the pins and perpendicular to the longitudinal axis.

10. The trocar seal of claim 8, wherein the receptacle and the pins are configured to support the angular movement of the reduction member in a plane aligned with the pins and perpendicular to the longitudinal axis.

11. The trocar seal of claim 8, wherein the connection between receptacle and the pins includes a bias, the bias configured to urge the reduction member to a first position wherein the hole is aligned with the passageway.

12. The trocar seal of claim 7, wherein the rim defining the hole in the interior portion has a proximal first portion and a distal second portion, the proximal first portion defining a conical shape and a distally decreasing inside diameter, the distal second portion defining a conical shape and a distally increasing inside diameter.

13. The trocar seal of claim 7, wherein at least a portion of the rim defining the opening is a cantilevered structure, the cantilevered structure including a bias, the bias configured to urge instruments towards the central longitudinal axis.

14. The trocar seal of claim 7, wherein the annular portion has a distal end and the valve assembly includes a proximal rim, the distal end of the annular portion being adapted for mounting on the proximal rim of the valve assembly, the proximal rim defining a portion of the passageway, the annular portion providing an unsealed proximal extension of the passageway between the rim of the valve assembly and the hole in the reduction member.

## Patentansprüche

1. Trokardichtung zur Verwendung mit einer Trokaranordnung, welche Dichtung umfasst:
ein Gehäuse (20), das zur Befestigung an einer Trokaranordnung (100) eingerichtet ist, wobei das Gehäuse eine äußere Wand, die eine Längsachse (X) definiert, und proximale (24) und distale (22) Enden enthält, wobei das Gehäuse eine innere Querwand (26) aufweist,
eine innere Dichtung (10), die innerhalb des Gehäuses allgemein mit der Längsachse fluchtend angeordnet ist, wobei die innere Dichtung eine Öffnung für eine abgedichtete Aufnahme eines länglichen Objekts, das darin eingeführt ist, aufweist, und
ein Verringerungselement (50), das an dem Gehäuse befestigt und relativ zu dem Gehäuse zwischen einer ersten und einer zweiten Position beweglich ist, wobei das Verringerungselement einen inneren Abschnitt aufweist, der eine Durchgangsöffnung (71) **dadurch** definiert, wobei die Durchgangsöffnung allgemein mit der Öffnung der inneren Dichtung fluchtend ist, wenn das Verringerungselement in seiner ersten Position ist, und frei von der Längsachse in der zweiten Position ist, wobei der innere Abschnitt in der ersten Position bemessen ist, um eine seitlich versetzte Bewegung des länglichen Objekts während einer Manipulation des länglichen Objekts zu minimieren, wenn das längliche Objekt in die innere Dichtung eingeführt und von dieser dichtend aufgenommen wird, wobei das Verringerungselement ferner einen ringförmigen Abschnitt (78) enthält, der distal von dem inneren Abschnitt angeordnet ist, wobei der ringförmige Abschnitt bemessen ist, um mit der Querwand des Gehäuses in Eingriff zu sein, um das Verringerungselement zu stützen, wenn es in seiner ersten Position ist.

2. Trokardichtung nach Anspruch 1, enthaltend eine äußere Dichtung, die umfangseitig in Bezug auf die innere Dichtung angeordnet und bemessen ist, um eine dichtende Kopplungsstelle zu definieren, um einen Durchgang von Insufflationsgasen durch das Gehäuse wesentlich zu minimieren.

3. Trokardichtung nach Anspruch 1, wobei das Verringerungselement einen Rand (72, 74) enthält, wobei der Rand die Durchgangsöffnung definiert, wobei der Rand einen proximalen ersten Abschnitt (72) und einen distalen zweiten Abschnitt (74) enthält, wobei der proximale erste Abschnitt eine konische Form und einen in distaler Richtung abnehmenden Innendurchmesser definiert, wobei der distale zweite Abschnitt eine konische Form und einen in distaler Richtung zunehmenden Innendurchmesser definiert.

4. Trokardichtung nach Anspruch 3, wobei zumindest ein Abschnitt (74) des Randes, der die Durchgangsöffnung definiert, eine auskragende Struktur ist, wobei die auskragende Struktur eine Vorspannung enthält, wobei die Vorspannung eingestellt ist, um Instrumente in Richtung der mittleren Längsachse zu drängen.

5. Trokardichtung nach Anspruch 1, wobei das Gehäuse einen proximalen Endabschnitt enthält, wobei der proximale Endabschnitt einen Rand (139) definiert, wobei der ringförmige Abschnitt des Verringerungselements zum Befestigen an dem Rand des proximalen Endabschnitts des Gehäuses eingerichtet ist, wobei der ringförmige Abschnitt eine unabgedichtete Verbindung zwischen dem Rand der Ventilanordnung und der Durchgangsöffnung in dem Verringerungselement schafft.

6. Trokardichtung nach Anspruch 1, wobei ein Feststellmechanismus an dem Verringerungselement angeordnet ist, wobei der Feststellmechanismus ein sich in distaler Richtung erstreckendes auskragendes Element enthält, das einen distalen Endabschnitt mit einer Protuberanz (23) aufweist, wobei sich die Protuberanz nach innen und radial erstreckt und eine allgemein flache, in proximale Richtung weisende Oberfläche aufweist, die zum Eingreifen mit einer Lippe (133) des Gehäuses eingerichtet ist, wobei die Lippe eine in distale Richtung weisende, allgemein flache Oberfläche aufweist.

7. Trokardichtung nach einem der vorstehenden Ansprüche, wobei
das Gehäuse eine röhrenförmige Wand enthält und an einem proximalen Ende einer Trokarventilanordnung angebracht ist, wobei die Ventilanordnung zumindest eine feste Dichtung einschließlich der inneren Dichtung aufweist und einen Längsdurchgang definiert, wobei der Längsdurchgang einen ersten Funktionsbereich definiert,
das Verringerungselement ein erstes Ende, ein gegenüber liegendes zweites Ende und einen inneren Abschnitt aufweist, wobei das erste Ende schwenkbar mit dem Gehäuse verbunden ist, wobei das zweite Ende einen Feststellmechanismus zum Eingreifen mit dem Gehäuse aufweist, wobei das Verringerungselement in der ersten Position über dem ersten Funktionsbereich des Durchgangs angeordnet ist, wobei das zweite Ende an dem Gehäuse festgestellt ist, wobei die schwenkbare Verbindung eine Bewegung ermöglicht und in der zweiten Position frei von dem ersten Funktionsbereich des Durchgangs angeordnet ist, wobei das Verringerungselement und das Gehäuse eine unabgedichtete Kopplungsstelle mit der Ventilanordnung schaffen,
ein Rand (13a) an dem inneren Abschnitt des Verringerungselements angeordnet ist, wobei der Rand eine Öffnung definiert, die mit dem Durchgang fluchtet, wobei die Öffnung einen zweiten Funktionsbereich definiert, der kleiner als der erste Funktionsbereich des Durchgangs ist.

8. Trokardichtung nach Anspruch 7, wobei das Gehäuse eine Aufnahme mit einem Paar Aussparungen (27) enthält und das erste Ende des Verringerungselements ein Paar gegenüber liegender linear fluchtender Pins (55) enthält, wobei die Pins zum Positionieren in den Aussparungen der Aufnahme gestaltet und bemessen sind und eine Bewegung des Verringerungselements ermöglichen.

9. Trokardichtung nach Anspruch 8, wobei die Aufnahme und die Pins gestaltet sind, um das Verschieben des Verringerungselements in einer mit den Pins fluchtenden Ebene und senkrecht zu der Längsachse zu unterstützen.

10. Trokardichtung nach Anspruch 8, wobei die Aufnahme und die Pins gestaltet sind, um die Winkelbewegung des Verringerungselements in einer mit den Pins fluchtenden Ebene und senkrecht zu der Längsachse zu unterstützen.

11. Trokardichtung nach Anspruch 8, wobei die Verbindung zwischen Aufnahme und den Pins eine Vorspannung enthält, wobei die Vorspannung eingestellt ist, um das Verringerungselement in eine erste Position zu drängen, wobei die Öffnung mit dem Durchgang fluchtet.

12. Trokardichtung nach Anspruch 7, wobei der Rand, der die Öffnung in dem inneren Abschnitt definiert, einen proximalen ersten Abschnitt und einen distalen zweiten Abschnitt aufweist, wobei der proximale erste Abschnitt eine konische Form und einen in distaler Richtung abnehmenden Innendurchmesser definiert, wobei der distale zweite Abschnitt eine konische Form und einen in distaler Richtung zunehmenden Innendurchmesser definiert.

13. Trokardichtung nach Anspruch 7, wobei zumindest ein Teil des Rands, der die Durchgangsöffnung definiert, eine auskragende Struktur ist, wobei die auskragende Struktur eine Vorspannung enthält, wobei die Vorspannung eingestellt ist, um Instrumente in Richtung der mittleren Längsachse zu drängen.

14. Trokardichtung nach Anspruch 7, wobei der ringförmige Abschnitt ein distales Ende aufweist und die Ventilanordnung einen proximalen Rand enthält, wobei das distale Ende des ringförmigen Abschnitts zur Befestigung an dem proximalen Rand der Ventilanordnung eingerichtet ist, wobei der proximale Rand einen Abschnitt des Durchgangs definiert, wobei der ringförmige Abschnitt eine unabgedichtete proximale Ausdehnung des Durchgangs zwischen dem Rand der Ventilanordnung und der Öffnung in dem Verringerungselement schafft.

## Revendications

1. Joint pour trocart pour utilisation avec un ensemble de trocart, ledit joint comprend:
un boîtier (20) apte à être monté sur un ensemble de trocart (100), le boîtier incluant une paroi extérieure définissant un axe longitudinal (X), et des extrémités proximale (24) et distale (22), le boîtier ayant une paroi transversale intérieure (26);
un joint interne (10) disposé dans le boîtier généralement aligné avec l'axe longitudinal, le joint interne ayant une ouverture pour la réception étanche d'un objet oblong introduit dans celle-ci; et
un élément de réduction (50) monté sur le boîtier et déplaçable relativement au boîtier entre des première et deuxième positions, l'élément de réduction ayant une portion intérieure définissant une ouverture (71) à travers celle-ci, l'ouverture étant généralement alignée avec l'ouverture du joint interne lorsque l'élément de réduction se trouve dans sa première position, et à distance de l'axe longitudinal dans la deuxième position, la portion intérieure dans ladite première position étant dimensionnée pour réduire à un minimum un mouvement latéral de décalage de l'objet oblong pendant la manipulation de l'objet oblong lorsque l'objet oblong est inséré dans et reçu d'une manière étanche par le joint interne, l'élément de réduction incluant en outre une portion annulaire (78) disposée distalement de la portion intérieure, la portion annulaire étant dimensionnée pour venir en prise avec la paroi transversale du boîtier pour supporter l'élément de réduction lorsqu'il se trouve dans sa première position.

2. Joint pour trocart selon la revendication 1, incluant un joint extérieur disposé périphériquement par rapport au joint intérieur et dimensionné pour définir une interface d'étanchéité afin de réduire sensiblement à un minimum le passage des gaz d'insufflation à travers le boîtier.

3. Joint pour trocart selon la revendication 1, où l'élément de réduction comprend un bord (72,74), le bord définissant l'ouverture, le bord incluant une première portion proximale (72) et une seconde portion distale (74), la première portion proximale définissant une forme conique et un diamètre intérieur diminuant distalement, la deuxième portion distale définissant une forme conique et un diamètre intérieur augmentant distalement.

4. Joint pour trocart selon la revendication 3, où au moins une portion (74) du bord définissant l'ouverture est une structure en porte-à-faux, la structure en porte-à-faux incluant un biais, le biais étant configuré pour solliciter des instruments vers l'axe longitudinal central.

5. Joint pour trocart selon la revendication 1, où le boîtier comprend une portion d'extrémité proximale, la portion d'extrémité proximale définissant un bord (139), la portion annulaire de l'élément de réduction étant apte à être montée sur le bord de la portion d'extrémité proximale du boîtier, la portion annulaire réalisant une connection non scellée entre le bord de l'ensemble de vanne et l'ouverture dans l'élément de réduction.

6. Joint pour trocart selon la revendication 1, où un mécanisme de verrouillage est positionné sur l'élément de réduction, le mécanisme de verrouillage incluant un élément en porte-à-faux s'étendant distalement ayant une portion d'extrémité distale avec une saillie (23), la saillie s'étendant vers l'intérieur et radialement et ayant une surface généralement plate orientée proximalement apte à venir en prise avec une lèvre (133) du boîtier, la lèvre ayant une surface généralement plate orientée distalement.

7. Joint pour trocart selon l'une quelconque des revendications précédentes, où le boîtier comprend une paroi tubulaire et est monté sur une extrémité proximale de l'ensemble de vanne de trocart, l'ensemble de vanne ayant au moins un joint fixe incluant le joint interne et définissant un passage longidinal, le passage longitudinal définissant une première zone opérable;
l'élément de réduction ayant une première extrémité, une deuxième extrémité opposée et une portion intérieure, la première extrémité étant reliée d'une manière pivotante au boîtier, la seconde extrémité ayant un mécanisme de verrouillage pour la mise en prise avec le boîtier, l'élément de réduction dans ladite première position étant positionné sur la première zone opérable du passage, la seconde extrémité étant verrouillée avec le boîtier, la connection pivotante permettant le mouvement et dans ladite seconde position étant positionnée à distance de la première zone opérable du passage, l'élément de réduction et le boîtier réalisant une interface non scellée avec l'ensemble de vanne;
un bord (13a) positionné sur la portion intérieure de l'élément de réduction, le bord définissant un trou aligné avec le passage, le trou définissant une deuxième zone opérable plus petite que la première zone opérable du passage.

8. Joint pour trocart selon la revendication 7, où le boîtier inclut un réceptacle ayant une paire d'encoches (27), et la première extrémité de l'élément de réduction inclut une paire d'axes opposés linéairement alignés (55), les axes étant configurés et dimensionnés pour le positionnement dans les encoches du réceptacle et permettant un mouvement de l'élément de réduction.

9. Joint pour trocart selon la revendication 8, où le réceptacle et les axes sont configurés pour supporter la translation de l'élément de réduction dans un plan aligné avec les axes et perpendiculaire à l'axe longitudinal.

10. Joint pour trocart selon la revendication 8, où le réceptacle et les axes sont configurés pour supporter le mouvement angulaire de l'élément de réduction dans un plan aligné avec les axes et perpendiculaire à l'axe longitudinal.

11. Joint pour trocart selon la revendication 8, où la connection entre le réceptacle et les axes comprend un biais, le biais étant configuré pour solliciter l'élément de réduction vers une première position où le trou est aligné avec le passage.

12. Joint pour trocart selon la revendication 7, où le bord définissant le trou dans la portion intérieure présente une première portion proximale et une seconde portion distale, la première portion proximale définissant une forme conique et un diamètre intérieur diminuant distalement, la seconde portion distale définissant une forme conique et un diamètre intérieur augmentant distalement.

13. Joint pour trocart selon la revendication 7, où au moins une portion du bord définissant l'ouverture est une structure en porte-à-faux, la structure en porte-à-faux incluant un biais, le biais étant configuré pour solliciter les instruments vers l'axe longitudinal central.

14. Joint pour trocart selon la revendication 7, où la portion annulaire présente une extrémité distale, et l'ensemble de vanne comprend un bord proximal, l'extrémité distale de la portion annulaire étant conçue pour être montée sur le bord proximal de l'ensemble de vanne, le bord proximal définissant une portion du passage, la portion annulaire réalisant une extension proximale non scellée du passage ente le bord de l'ensemble de vanne et le trou dans l'élément de réduction.
